# FASCICULE DE BREVET EUROPEEN

(11) **EP 3 063 127 B1**
(45) Date de publication et mention de la délivrance du brevet: **13.12.2017**
(21) Numéro de dépôt: 14809519.3
(22) Date de dépôt: 29.10.2014
(51) Int. Cl.: C07C 327/18, C25B 11/04, H01L 51/00

(54) **MATERIAU D'ELECTRODE POUR BATTERIE ORGANIQUE COMPRENANT DES DÉRIVÉS DE L'ACIDE BENZENE-BISDITHIOÏQUE**
MATERIAL FÜR EINE ELEKTRODE EINER ORGANISCHEN BATTERIE MIT BENZOL-BIS(DITHIO)SÄURE-DERIVATEN
MATERIAL FOR AN ELECTRODE OF AN ORGANIC BATTERY COMPRISING BENZENE-BIS(DITHIOIC) ACID DERIVATIVES

(30) Priorité: 30.10.2013 FR 1360611
(43) Date de publication de la demande: 07.09.2016
(73) Titulaire: Commissariat à l'Energie Atomique et aux Energies Alternatives, 75015 Paris (FR)
(72) Inventeur: GUTEL, Thibaut, 38113 Veurey-voroize (FR); DUBOIS, Lionel, 73460 Verrens-arvey (FR); IORDACHE, Adriana, 38000 Grenoble (FR)
(74) Mandataire: Nony
(86) Numéro de dépôt international: PCT/IB2014/065682
(87) Numéro de publication internationale: WO 2015/063697

(56) Documents cités:
- US-A1- 2012 258 360
- YANGJUN XING ET AL: "Optimizing Single-Molecule Conductivity of Conjugated Organic Oligomers with Carbodithioate Linkers", JOURNAL OF THE AMERICAN CHEMICAL SOCIETY, vol. 132, no. 23, 16 juin 2010 (2010-06-16), pages 7946-7956, XP055127102, ISSN: 0002-7863, DOI: 10.1021/ja909559m
- ELEFTHERIA NEOFOTISTOU ET AL: "Novel Coordination Polymers Based on the Tetrathioterephthalate Dianion as the Bridging Ligand", INORGANIC CHEMISTRY, vol. 46, no. 21, 1 octobre 2007 (2007-10-01), pages 8487-8489, XP055127126, ISSN: 0020-1669, DOI: 10.1021/ic701414v
- M. ARMAND ET AL: "Conjugated dicarboxylate anodes for Li-ion batteries", NATURE MATERIALS, vol. 8, no. 2, 1 février 2009 (2009-02-01) , pages 120-125, XP055127282, ISSN: 1476-1122, DOI: 10.1038/nmat2372

## Description

La présente invention concerne le domaine des batteries secondaires au lithium, sodium ou potassium. Elle vise plus particulièrement à proposer un nouveau matériau actif d'électrodes pour ces batteries secondaires et notamment pour des batteries lithium-ion.

Les batteries au lithium sont de plus en plus utilisées comme sources d'énergie autonome, en particulier dans les équipements portables, où ils remplacent progressivement les accumulateurs nickel-cadmium (NiCd) et nickel-hydrure métallique (NiMH). Cette évolution s'explique par l'amélioration continue des performances des accumulateurs au lithium, leur conférant ainsi des densités d'énergie nettement supérieures à celles proposées par les filières NiCd et NiMH. Les batteries au lithium trouvent de multiples applications, notamment dans les nouvelles technologies de l'information et de la communication (NTIC), des dispositifs médicaux, les véhicules électriques, le stockage de l'énergie de cellules photovoltaïques, etc.

Ces générateurs électrochimiques au lithium fonctionnent classiquement sur le principe d'insertion ou de désinsertion (ou intercalation-désintercalation) du lithium sur au moins une électrode. En particulier, dans un accumulateur lithium-ion, les cations Li⁺ font ainsi des allers-retours entre les électrodes, respectivement positive et négative, à chaque charge et décharge de l'accumulateur. Le matériau actif de l'électrode positive est capable de libérer des ions lithium au moment de la charge et d'incorporer des ions lithium au moment de la décharge.

D'une manière générale, les composés actifs d'électrodes utilisés dans les accumulateurs commerciaux sont, pour l'électrode positive, des composés lamellaires tels que LiCoO₂, LiNiO₂ et mixtes Li(Ni, Co, Mn, Al)O₂ ou des composés de structure spinelle de compositions proches de LiMn₂O₄. L'électrode négative est généralement du carbone (graphite, coke, etc.) ou éventuellement le spinelle Li₄Ti₅O₁₂ ou un métal formant un alliage avec le lithium (Sn, Si, etc.).

Pour répondre plus particulièrement aux nouveaux marchés de l'automobile hybride et électrique ou du solaire photovoltaïque, les contraintes de coût, de volume de production et de performances en puissance imposent la recherche de nouveaux matériaux actifs d'électrode.

Dans cette optique, des polymères ont déjà été proposés, par exemple dans les documents WO 2009/043729, WO 2009/127674 ou EP 1 244 168, comme matériaux actifs, pour leur propriété de conduction de l'électricité, en remplacement ou en complément de conducteurs électroniques classiques tels que le noir de carbone. Ils sont principalement utilisés pour leur facilité de mise en oeuvre, comparée aux suspensions de particules conductrices, et permettent d'améliorer la percolation électrique des matériaux actifs d'électrode.

Les polymères les plus couramment employés sont des polyanilines, des polypyrroles, des composés conducteurs de protons contenant au moins un hétérocycle avec un atome d'azote, ou encore des polymères présentant sur leur chaîne un composant portant un pont disulfure.

A titre d'exemple, le document US 7,651,647 propose la mise en oeuvre d'un mélange d'un matériau actif tel que l'oxyde de vanadium-argent ou du carbone fluoré et d'un polymère conducteur choisi parmi la polyaniline, le polydioxythiophène et leur combinaison, pour la fabrication d'une électrode positive d'une cellule électrochimique.

On peut encore citer le document JP 2008-192452 qui décrit l'utilisation, comme matériaux actifs d'électrodes, de polymères conducteurs formés à partir de monomères présentant des cycles hétéroaromatiques à cinq chaînons, en particulier des polythiophènes porteurs de radicaux TEMPO ou dérivés. Ces polymères sont synthétisés par greffage du motif TEMPO sur les monomères, suivi de la polymérisation.

Toutefois, l'utilisation de ces polymères greffés dans des batteries secondaires au lithium n'est pas totalement satisfaisante. Ainsi, elle nécessite la mise en oeuvre conjointe d'importantes quantités de conducteurs électroniques classiques, tels que du noir de carbone, généralement entre 20 % et 70 % en poids, afin de permettre l'extraction des électrons du matériau actif. Par ailleurs, l'ajout de telles quantités de conducteurs électroniques se fait malheureusement au détriment de la densité d'énergie massique ou volumique de l'électrode, et rend toute application industrielle fortement onéreuse. Enfin, l'utilisation de liants complémentaires est nécessaire pour assurer l'adhérence du matériau polymère sur le collecteur de courant et une bonne tenue mécanique de l'électrode

Récemment, les documents US 2009/181309, US 2009/152509 et US 2005/239917 ont proposé des composés carbonyles comme matériaux de cathode pour les batteries au lithium.

Néanmoins, bien que ces matériaux soient avantageux en termes d'énergie et de densité de puissance, ils présentent de nombreux problèmes de transport électronique et ionique de l'électrode, et de stabilité chimique, thermique et électrochimique du matériau. En outre, ces matériaux possèdent un potentiel rédox inadéquat.

La présente invention vise à proposer de nouveaux composés utilisables comme matériau d'électrode pour des batteries secondaires au lithium, sodium ou potassium, de préférence des batteries secondaires au lithium, et permettant notamment de pallier les inconvénients précités.

Plus particulièrement, elle concerne, selon un premier de ses aspects, l'utilisation comme matériau actif d'électrode d'un composé comprenant au moins une entité de formule (I) : 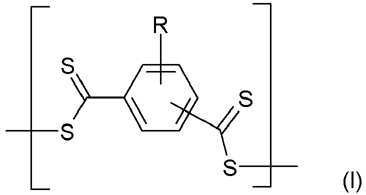 dans laquelle le groupement phénylique est substitué par un à quatre substituant(s) R, identique(s) ou différent(s), choisi(s) parmi un atome d'hydrogène, un atome d'halogène choisi parmi le fluor, le chlore, le brome ou l'iode, un groupement -C(=S)-S⁻C⁺, un groupement -O⁻C⁺, un groupement -S⁻C⁺, avec C⁺ étant un cation alcalin choisi parmi Li⁺, Na⁺ et K⁺, de préférence Li⁺; un radical (C₁-C₁₂)alkyle, un radical (C₂-C₁₂)alkényle, un radical (C₆-C₁₄)aryle ou hétéroaryle ; ou deux substituants R vicinaux pouvant le cas échéant être liés entre eux pour former ensemble un cycle de 3 à 7 chaînons incluant éventuellement un autre hétéroatome choisi parmi N, O ou S ;
à l'état de base ou de sel ;
ainsi que ses formes tautomères.

De tels composés s'avèrent particulièrement avantageux comme matériau actif d'électrodes pour des batteries secondaires au lithium, au sodium ou au potassium, de préférence au lithium.

La mise en oeuvre des composés selon l'invention comme matériau actif d'électrode s'avère avantageuse à plusieurs titres.

Tout d'abord, les électrodes formées selon l'invention présentent une bonne flexibilité et légèreté, propriétés particulièrement recherchées dans le cadre des systèmes portables.

Elles présentent une très bonne stabilité chimique, thermique et électrochimique.

Elles possèdent des propriétés de transport électronique et ionique améliorées, notamment par rapport à celles mettant en oeuvre les composés carbonyles décrits dans les documents US 2009/181309, US 2009/152509 et US 2005/239917, et possèdent également un potentiel redox élevé.

Les documents US-A-2012/258,360 et Nature Materials Vol 8(2) pages 120-125 (2009) décrivent l'utilisation du composé téréphtalate de di-lithium comme matériau actif d'électrode. De plus, contrairement aux électrodes classiques à base de matériaux inorganiques d'insertion pulvérulents, les électrodes comprenant les composés de l'invention peuvent être mises en forme selon des techniques classiques d'enduction ou d'impression, ou toute autre technique de mise en oeuvre de matériaux organiques en solution (techniques de coating comme la tournette, le deep-coating, le spray, le drop casting) ou à l'état fondu (technique d'extrusion, d'injection, de rotomoulage, d'extrusion soufflage...).

D'autres caractéristiques, variantes et avantages des composés selon l'invention, de leur préparation et de leur mise en oeuvre ressortiront mieux à la lecture de la description, des exemples et figures qui vont suivre, donnés à titre illustratif et non limitatif de l'invention.

### Sujet de l'invention

Comme précisé ci-dessus, les composés utilisés selon l'invention comprennent au moins une entité de formule (I) : 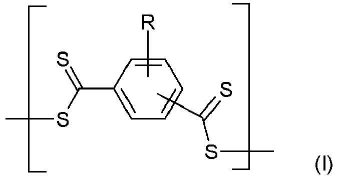 dans laquelle le groupement phénylique est substitué par un à quatre substituant(s) R, identique(s) ou différent(s), choisi(s) parmi un atome d'hydrogène, un atome d'halogène choisi parmi le fluor, le chlore, le brome ou l'iode, un groupement -C(=S)-S⁻C⁺, un groupement -O⁻C⁺, un groupement -S⁻C⁺, avec C⁺ étant un cation alcalin choisi parmi Li⁺, Na⁺ et K⁺, de préférence Li⁺; un radical (C₁-C₁₂)alkyle, un radical (C₂-C₁₂)alkényle, un radical (C₆-C₁₄)aryle ou hétéroaryle ; ou deux substituants R vicinaux pouvant le cas échéant être liés entre eux pour former ensemble un cycle de 3 à 7 chaînons incluant éventuellement un autre hétéroatome choisi parmi N, O ou S ;
à l'état de base ou de sel ;
ainsi que ses formes tautomères.

Dans le cadre de la présente invention :
- « Cₜ-C_{z}, où t et z peuvent prendre les valeurs de 1 à 14 », désigne une chaine carbonée pouvant avoir de t à z atomes de carbone, à l'image d'une chaine carbonée en C₁-C₁₂ ;
- un atome d'halogène désigne un fluor, un chlore, un brome ou un iode ;
- un alkyle désigne un groupe aliphatique saturé linéaire, ramifié ou cyclique, comprenant de 1 à 12 atomes de carbone. A titre d'exemples, on peut citer les groupes méthyle, éthyle, propyle, isopropyle, butyle, isobutyle, tertbutyle, cyclohexyle, cyclopentyle, etc. ;
- un alkényle désigne un groupe aliphatique mono- ou polyinsaturé, linéaire, ramifié ou cyclique, comprenant de 2 à 12 atomes de carbone, comprenant une ou plusieurs insaturations éthyléniques. A titre d'exemples, on peut citer les groupes éthylène, 1-méthyléthylène, propylène, cyclohexadiène, etc.;
- un aryle désigne un groupe aromatique mono-, bi- ou tricyclique comprenant entre 6 et 14 atomes de carbones. A titre d'exemples de groupes aryles, on peut citer les groupes phényle, naphtyle, etc. ;
- un hétéroaryle désigne un groupe hétérocyclique mono-, bi- ou tricyclique aromatique de 5 à 14 chaînons contenant de 1 à 8 hétéroatomes choisis parmi l'oxygène, le soufre et l'azote. A titre d'exemples de groupes hétéroaryles, on peut citer les groupes pyridine, pyrazine, pyrimidine, pyrazole, oxadiazole, thiazole, imidazole, benzothiophène, quinoline, indole, etc. ;
- un cycle de 3 à 7 chaînons désigne un groupe cyclique non aromatique, insaturé ou saturé, de 3 à 7 chaînons, comprenant éventuellement un ou plusieurs hétéroatomes choisi parmi l'oxygène, le soufre et l'azote. A titre d'exemples de cycles de 3 à 7 chaînons, on peut citer les groupes cyclopropyle, cyclopropene, cyclobutyle, cyclobutene, cyclopentyle, cyclopentene, cyclohexyle, azétidinyle, pyrrolidinyle, pipéridinyle, morpholinyle, thiomorpholinyle, pipérazinyle, homopipérazinyle, dihydro-oxazolyle, dihydro-thiazolyle, dihydro-imidazolyle, dihydro-pyrrolyle ou tétrahydro-pyridinyle, etc.

La présente invention concerne également les formes tautomériques des composés comprenant une entité de formule (I) selon l'invention.

On entend par une « une forme tautomère », un isomère de constitution dont la structure diffère par la position d'un atome, par exemple un atome d'hydrogène, et d'une ou de plusieurs liaisons multiples. Deux formes tautomères sont capables de se transformer facilement et réversiblement l'une en l'autre.

Les composés comprenant au moins une entité de formule (I) peuvent exister à l'état de bases ou de sels. De tels sels font également partie de l'invention.

Par exemple, les sels peuvent être des sels de cation alcalin, alcalino-terreux ou de métaux de transition, tel que par exemple l'alcoolate de lithium/Na, le sulphonate de Li/Na, le dialkyleamide de Li/Na, etc. Il peut également s'agir de sel de cation organique tel que les sels ammonium, phosphonium ou sulfonium.

Selon un mode de réalisation particulièrement préféré, les composés comprenant au moins une entité de formule (I) sont choisis parmi les composés de formule (II) : 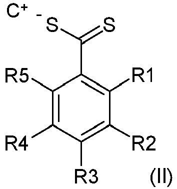 dans laquelle :
- C⁺ est un cation alcalin choisi parmi Li⁺, Na⁺ et K⁺, de préférence Li⁺ ;
- au moins l'un des substituants R₁, R₂, R₃, R₄ ou R₅ représente un groupement -C(=S)-S⁻, C⁺, les autres, identiques ou différents, représentant un groupe R choisi parmi un atome d'hydrogène, un atome d'halogène choisi parmi le fluor, le chlore, le brome ou l'iode, un groupement -C(=S)-S⁻C⁺, un groupement -O⁻C⁺, un groupement -S⁻C⁺, C⁺ étant tel que défini précédemment ; un radical (C₁-C₁₂)alkyle, un radical (C₂-C₁₂)alkényle, un radical (C₆-C₁₄)aryle ou hétéroaryle ; ou deux substituants R vicinaux pouvant le cas échéant être liés entre eux pour former ensemble un cycle de 3 à 7 chaînons incluant éventuellement un autre hétéroatome choisi parmi N, O ou S ;
   à l'état de base ou de sel ;
   ainsi que ses formes tautomères.

Selon un de ses aspects, la présente invention concerne plus particulièrement des composés de formule (IIa) suivante : 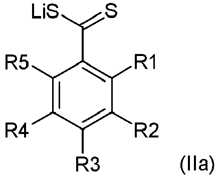 dans laquelle :
- au moins l'un des substituants R₁, R₂, R₃, R₄ ou R₅ représente un groupement -C(=S)-SLi ; les autres, identiques ou différents, représentant un groupe R choisi parmi un atome d'hydrogène, un atome d'halogène choisi parmi le chlore, le brome ou l'iode, un groupement -C(=S)-SLi, un groupement -OLi, un groupement -SLi, un radical (C₁-C₁₂)alkyle, un radical (C₂-C₁₂)alkényle, un radical (C₆-C₁₄)aryle ou hétéroaryle ; ou deux substituants R vicinaux pouvant le cas échéant être liés entre eux pour former ensemble un cycle de 3 à 7 chaînons incluant éventuellement un autre hétéroatome choisi parmi N, O ou S ;
   à l'état de base ou de sel ;
   ainsi que ses formes tautomères.

De préférence, dans les composés selon l'invention, R₃ représente un groupement -C(=S)-S⁻C⁺, dans lequel C⁺ désigne un cation alcalin choisi parmi Li⁺, Na⁺ et K⁺, de préférence Li⁺ et R₁, R₂, R₄ et R₅, identiques ou différents, représentent un substituant R tel que défini ci-dessus.

Selon un mode de réalisation particulier, R représente un atome d'hydrogène, un groupement -C(=S)-S⁻C⁺, un groupement -O⁻C⁺ ou un groupement -S⁻C⁺, avec C⁺ étant un cation alcalin choisi parmi Li⁺, Na⁺ et K⁺, de préférence Li⁺.

De préférence, R représente un atome d'hydrogène.

Plus particulièrement, il peut s'agir du composé (1,4-benzene)-bisdithioate de bis-lithium de formule : 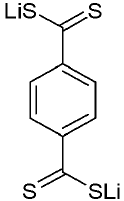 ou sa forme tautomère.

Les produits comprenant au moins une entité de formule (I) tels que définis ci-dessus peuvent être préparés par les méthodes générales connues de l'homme du métier.

Par ailleurs, les composés de formule (II) selon l'invention peuvent être préparés selon le procédé de préparation comprenant au moins :
(a) la réaction d'éthanolate de lithium, de sodium ou de potassium avec du soufre ; et
(b) la mise en contact du mélange réactionnel obtenu à l'issue de l'étape (a) avec un composé de formule (III) : 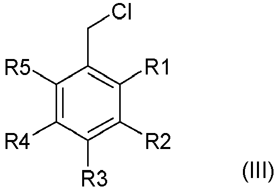 dans laquelle :
   - au moins l'un des substituants R₁, R₂, R₃, R₄ ou R₅ représente un groupement -CH₂-Cl, les autres, identiques ou différents, représentant un groupe R choisi parmi un atome d'hydrogène, un atome d'halogène choisi parmi le fluor, le chlore, le brome ou l'iode, un groupement -CH₂-Cl, un groupement -O⁻C⁺, un groupement -S⁻C⁺, avec C⁺ étant un cation choisi parmi Li⁺, Na⁺ et K⁺, un radical (C₁-C₁₂)alkyle, un radical (C₂-C₁₂)alkényle, un radical (C₆-C₁₄)aryle ou hétéroaryle ; ou deux substituants R vicinaux pouvant le cas échéant être liés entre eux pour former ensemble un cycle de 3 à 7 chaînons incluant éventuellement un autre hétéroatome choisi parmi N, O ou S ;
      dans des conditions propices à la formation du composé de formule (II).

Selon une variante particulière de ce procédé, l'étape (a) du procédé est effectuée à une température comprise entre 25°C et 150°C pendant une durée variant de 1 heure à 48 heures.

Bien entendu, il appartient à l'homme du métier d'ajuster les conditions de synthèse pour obtenir les composés selon l'invention.

Les composés selon l'invention sont généralement obtenus sous forme d'un solide qui se prête à une utilisation telle quelle ou par exemple en solution.

### Matériau actif d'électrode

Comme précisé précédemment, le composé selon l'invention est particulièrement avantageux comme matériau actif d'électrode.

Par « matériau actif d'électrode », on entend au sens de l'invention un matériau d'insertion/désinsertion d'un cation C⁺ (Li⁺, Na⁺ ou K⁺) d'une électrode d'un générateur électrochimique. Plus particulièrement, le matériau actif de l'électrode positive est capable de libérer des ions C⁺ au moment de la charge et d'incorporer des ions C⁺ au moment de la décharge du générateur électrochimique. Inversement, le matériau actif de l'électrode négative est capable d'incorporer des ions C⁺ au moment de la charge et de libérer des ions C⁺ au moment de la décharge du générateur électrochimique.

Ainsi, la présente invention concerne l'utilisation d'un composé tel que défini précédemment comme matériau actif d'électrode.

Selon un autre de ses aspects, l'invention concerne également un matériau actif d'électrode comprenant au moins un composé tel que défini précédemment.

Le matériau d'électrode peut être préparé sous la forme d'une poudre dispersé ou en solution.

Le matériau d'électrode peut comprendre un composé ou mélange de composés selon l'invention. Il peut comprendre, outre le ou lesdits composés de l'invention, un ou plusieurs composés additionnels classiquement mis en oeuvre, comme par exemple un liant ou un additif conducteur.

Avantageusement, ce matériau est immobilisé au niveau de l'électrode.

### Electrode

Selon encore un autre de ses aspects, l'invention concerne une électrode formée en tout ou partie d'un matériau d'électrode tel que décrit précédemment.

Elle vise plus particulièrement une électrode formée en tout ou partie d'un matériau actif comprenant au moins un composé tel que défini précédemment et au moins un additif conducteur électronique.

De préférence, le matériau d'électrode représente de 10 % à 95 % en poids du poids total de l'électrode, en particulier plus de 40 % en poids, et plus particulièrement de 40 % à 80 % en poids, par rapport au poids total de ladite électrode.

Une électrode selon l'invention peut être utilisée comme électrode positive ou comme électrode négative, en particulier d'un générateur au lithium.

De manière classique, une électrode selon l'invention peut comporter un collecteur de courant sur laquelle est appliqué ledit matériau actif d'électrode, notamment *via* les techniques développées ci-dessous.

Par exemple, de l'aluminium ou de l'acier inoxydable peuvent être utilisés comme collecteur de courant pour une électrode positive ; et du cuivre, du nickel ou de l'acier, traités en une feuille découpée, un métal en mousse ou une plaque de feuille laminé, par exemple, peuvent être utilisés comme collecteur de courant pour une électrode négative.

Avantageusement, une électrode selon l'invention peut être exempte de collecteur de courant.

Selon un mode de réalisation particulier, l'électrode peut comprendre en outre un ou plusieurs additif(s) conducteur(s) électronique(s).

Le ou lesdits additifs conducteurs électroniques peuvent être choisis parmi des fibres de carbone, du noir de carbone, des nanotubes de carbone, du graphène et leurs analogues.

Selon un mode de réalisation préféré, le ou lesdits additifs conducteurs électroniques peuvent être présents en une quantité inférieure ou égale à 60 % en poids par rapport au poids total de ladite électrode, de préférence inférieure ou égale à 40 % en poids, et plus particulièrement inférieure ou égale à 20 % en poids, par rapport au poids total de ladite électrode.

Selon un mode de réalisation particulier, l'électrode peut être exempte d'additif conducteur électronique.

Selon un autre mode de réalisation particulier, l'électrode peut comprendre en outre un ou plusieurs liant(s).

De tels liants peuvent être avantageusement choisi(s) parmi des liants fluorés, en particulier parmi le polytétrafluoroéthylène, le polyfluorure de vinylidène, les polymères dérivés de carboxyméthylcellulose, les polysaccharides et les latex notamment de type caoutchouc styrène-butadiène (SBR ou en langue anglaise « styrene-butadiene rubber »).

Selon un mode de réalisation préféré, le ou lesdits liants peuvent être présents en une quantité inférieure ou égale à 40 % en poids, par rapport au poids total de l'électrode, de préférence inférieure ou égale à 20 % en poids, notamment inférieure ou égale à 10 % en poids, et plus particulièrement inférieure ou égale à 5 % en poids, par rapport au poids total de l'électrode.

En particulier, une électrode selon l'invention peut comprendre moins de 40 % en poids de liants fluorés, en particulier moins de 20 % en poids de liants fluorés, par rapport au poids total de l'électrode.

Selon une variante de réalisation particulière, une électrode selon l'invention est exempte de liant.

Une électrode peut en outre comprendre d'autres additifs couramment mis en oeuvre pour des électrodes de batteries secondaires.

### Procédé de préparation d'électrode

Une électrode selon l'invention peut être préparée suivant différentes techniques.

Selon une première variante de réalisation, l'électrode selon l'invention peut être formée par un procédé de préparation, comprenant au moins les étapes consistant en :
(i) disposer d'un mélange formé d'au moins un composé tel que défini précédemment, d'une phase liquide en particulier comprenant un ou plusieurs solvants organiques ou aqueux, et éventuellement d'un ou plusieurs additif(s) conducteur(s) électronique(s) et/ou liant(s) ; et
(ii) déposer ledit mélange de l'étape (i) par enduction ou par une technique d'impression sur un substrat de base ou par extrusion ou par colaminage.

La dispersion de l'étape (i), lorsqu'elle est aqueuse, peut également comporter un épaississant, par exemple de type carboxy méthylcellulose, hydroxypropyle méthylcellulose, et/ou un surfactant et/ou un sel (LiOH par exemple). Une telle dispersion est également couramment appelée « encre ». L'encre peut par exemple être déposée selon l'étape (ii) sur un collecteur de courant, tel qu'une feuille métallique, par exemple en aluminium ou en cuivre. Le dépôt de l'encre peut par exemple être réalisé par flexographie, héligravure, sérigraphie, jet d'encre ou spray. L'homme du métier est à même d'ajuster les conditions de mise en oeuvre de ces différentes techniques.

En ce qui concerne le substrat de base, il peut s'agir d'un film polymérique de type polyéthylène ou polypropylène. L'étape (ii) est alors suivie d'une étape ultérieure (iii) de décollage dudit film polymérique, pour former une électrode autosupportée.

Selon une seconde variante de réalisation, une électrode conforme à l'invention peut être formée par électropolymérisation *in situ* en solution. Ainsi, une couche polymérique formée en tout ou partie d'entités de formule (I) selon l'invention peut être générée par électropolymérisation de monomères fonctionnalisés par des entités de formule (I). Cette électrode peut être réalisée directement sur un collecteur de courant, tel que, par exemple, une feuille d'aluminium, de nickel, de cuivre, d'acier ou de carbone, le cas échéant en présence de conducteurs électroniques additionnels.

L'invention concerne ainsi, selon un autre de ses aspects, un procédé de préparation d'une électrode telle que définie précédemment, comprenant la formation d'un polymère par électropolymérisation *in situ* sur un collecteur de courant, de monomères fonctionnalisés par des entités de formule (I) telles que décrites précédemment.

Les monomères à fonctionnaliser peuvent notamment être choisis dans la famille des pyrroles, thiophènes, fluorènes, carbazoles, anilines, phénylènes, isothionaphtènes, acétylènes, phénylènevinylènes et leurs mélanges.

Selon une variante particulièrement préférée, l'électropolymérisation a lieu directement au sein d'une cellule électrochimique au lithium, par exemple une cellule électrochimique au lithium. Dans cette configuration, l'électropolymérisation a lieu lors du premier cycle de charge (ou décharge) de la cellule et l'électrode se forme *in situ* sur le collecteur de l'électrode positive (ou négative respectivement).

Plus particulièrement, le procédé de formation de l'électrode par électropolymérisation *in situ* peut comprendre au moins les étapes consistant en :
(i) introduire lesdits monomères fonctionnalisés par des entités de formule (I) sous forme de poudre dans l'électrolyte d'une cellule électrochimique au lithium, au sodium ou au potassium, ou mettre en solution lesdits monomères fonctionnalisés dans l'électrolyte d'une cellule électrochimique au lithium, au sodium ou au potassium ; et
(ii) effectuer au moins un cycle de charge de la cellule pour former une électrode positive, ou au moins un cycle de décharge de la cellule pour former une électrode négative.

Dans le cas particulier d'une cellule électrochimique au lithium, la solution d'électrolyte peut être choisie parmi les électrolytes classiquement mis en oeuvre pour des batteries au lithium, au travers desquels les cations Li⁺ ont la possibilité de migrer. L'électrolyte peut, par exemple, être constitué par un sel comportant au moins le cation Li⁺. Le sel est, par exemple, choisi parmi LiClO₄, LiAsF₆, LiPF₄, LiBF₄, LiR_{F}SO₃, LiCH₃SO₃, LiN(R_{F}SO₂)₂, LiN(R_{F}SO₂)₃; R_{F} étant choisi parmi un atome de fluor et un groupement perfluoroalkyle comportant entre 1 et 8 atomes de carbone. Le sel est, de préférence, dissous dans un solvant polaire aprotique tel que le carbonate d'éthylène, le carbonate de propylène, le carbonate de diméthyle, le carbonate d'éthylméthyle ; et il peut être supporté par un élément séparateur disposé entre les première et seconde électrodes, l'élément séparateur étant alors imbibé d'électrolyte.

D'autres variantes de réalisation d'une électrode selon l'invention sont également envisageables, notamment :
- par mise en oeuvre des composés de l'invention en solution : solubilisation du matériau actif selon l'invention dans un solvant, éventuellement en présence de conducteurs électroniques additionnels, et dépôt de la solution par une technique classique, par exemple choisie parmi les techniques d'impression (flexographie, héligravure, sérigraphie, jet d'encre, spray ...), drop casting, dip-coating, tournette. La mise en oeuvre en solution facilite le dépôt sur des collecteurs souples du type polymère avec un dépôt métallique ou tissus carbonés ; ou encore
- par mise en oeuvre des composés de l'invention à l'état fondu : injection, extrusion, rotomoulage par exemple, éventuellement en présence de conducteurs électroniques additionnels. Dans le cas de l'extrusion, l'extrudat se présentera sous forme de jonc qui sera appliqué sur le collecteur de courant par compression (presse isostatique, calandrage, ...) ou préférentiellement sous forme de film qui sera déposé à l'état fondu directement sur un collecteur conducteur électrique (Al, de Ni, de Cu, acier, carbone...).

### Batterie secondaire au lithium, au sodium ou au potassium

Comme évoqué précédemment, la présente invention se rapporte à une batterie secondaire au lithium, au sodium ou au potassium, comprenant une électrode selon l'invention.

De préférence, il s'agit d'une batterie secondaire au lithium.

Il peut s'agir d'une batterie lithium-ion, lithium-polymère, lithium-soufre, lithium-air ou supercondensateur, et de préférence une batterie lithium-ion.

Le reste de la batterie peut être formée selon des méthodes conventionnelles.

De manière générale, les batteries lithium-ion présentent une architecture avec deux électrodes (une électrode positive et une électrode négative), toutes les deux revêtues sur un collecteur de courant conducteur électrique, disposées de part et d'autre d'un séparateur organique ou inorganique. Les deux techniques de montage de cette architecture actuellement les plus utilisées sont le bobinage (enroulement des différents constituants dans une géométrie cylindrique ou prismatique) et le stack (empilement couche par couche des différents éléments). Bien entendu, d'autres techniques de montage pour former une batterie sont envisageables, telles que les techniques de l'impression.

L'électrode selon l'invention peut constituer l'électrode positive ou l'électrode négative de la batterie. De préférence, elle constituera l'électrode négative.

Dans le texte, les expressions « compris entre ... et ... » et « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

L'invention va maintenant être décrite au moyen des figures et exemples suivants donnés bien entendu à titre illustratif et non limitatif de l'invention.

### FIGURES

Figure 1 : Courbes de voltampérométrie cyclique du BBDTLi₂ : en solution sur électrode de Pt (2 mm) (Figure 1A) et sur carbone vitreux (3 mm) (Figure 1B) Figure 2 : Courbes de voltampérométrie cyclique du BBDTPLi₂ en pile bouton
Figure 3 : Courbes de capacité-potentiel du BBDTLi₂ en pile bouton
Figures 4 à 6 : Courbes de cyclage du BBDTLi₂ à différents régimes
Figure 7 : Spectre UV-Vis enregistré pendant l'électrolyse de BBDTLi₂
Figure 8 : Courbes de capacité-potentiel du TPLi₂ (non conforme à l'invention) en pile bouton
Figures 9 et 10 : Courbes de cyclage du TPLi₂ (non conforme à l'invention) à différents régimes
Figure 11 : Comparaison de la capacité au régime C/10 pour le BBDTLi₂ et le TPLi₂
Figure 12 : Comparaison de la capacité en fonction du nombre de cycles pour le BBDTLi₂ (régime C/10) et le TPLi₂ (régime C/50).

### EXEMPLES

### EXEMPLE 1

### Préparation du (1,4-benzene)-bisdithioate de bis-lithium (BBDTLi₂)

Une solution d'éthanolate de lithium est préparée par ajout de 10 mL d'éthanol anhydre sur 0,16 g de lithium métallique, dans une fiole à brome.

Cette solution est ensuite ajoutée goutte-à-goutte à 5,86 g de soufre cristallisé dans 90 mL d'éthanol anhydre.

Le mélange réactionnel est maintenu à reflux pendant approximativement 2 heures.

Un équivalent de α,α'-dichlorure de *p*-xylène (1 g) est ensuite ajouté sous forme solide au mélange réactionnel et porté à reflux pendant 20 heures.

Après refroidissement à température ambiante, le mélange brut est filtré afin d'éliminer l'excès de soufre, puis le filtrat est concentré sous pression réduite et à nouveau filtré.

La solution est ensuite évaporée à sec pour donner un solide rouge.

Le solide est lavé en abondance avec du toluène, du cyclohexane et du dichlorométhane.

Le solide résiduel brut est purifié par recristallisation lente à température ambiante dans un mélange éther/cyclohexane.

La structure du composé (1,4-benzene)-bisdithioate de bis-lithium obtenu est caractérisée par diffraction des rayons X sur monocristal. Les données cristallines obtenues sont détaillées dans le tableau 1 ci-dessous.

**Tableau 1**

| Composé | (1,4-benzene)-bisdithioate de bis-lithium (BBDTLi₂) | |
|---|---|---|
| Formule empirique | C₈H₁₆Li₂O₆S₄ | |
| Masse atomique | 350.33 | |
| Température | 150(2) K | |
| Longueur d'onde | 0.71073 A | |
| Système cristallin | Monoclinique | |
| Groupe spatial | P 1 21/c 1 | |
| Dimensions de maille élémentaire | a = 8.0373(5) A | alpha = 90° |
| | b = 16.5938(11) A | beta = 104.367(8)° |
| | c = 6.0809(6) A | gamma = 90° |
| Volume, Z | 785.65(10) A³, 2 | |
| Densité (calculé) | 1.481 g/cm³ | |
| Coefficient d'absorption | 0.618 mm⁻¹ | |
| F(000) | 364 | |
| Taille du cristal | 0.40 x 0.05 x 0.02 mm | |

### EXEMPLE 2

### Voltampérométrie cyclique du BBDTLi₂ en solution

Une solution à 1 mM de (1,4-benzene)-bisdithioate de bis-lithium dans l'acétonitrile (AN) contenant 0,1 M hexafluorophosphate de tétrabutylammonium (TBAPF₆) est préparée. Les courbes de voltampérometrie cyclique sont mesurées en balayant le potentiel entre 1 et 4 V vs Li⁺/Li⁰ à une vitesse de 0,1 V/s.

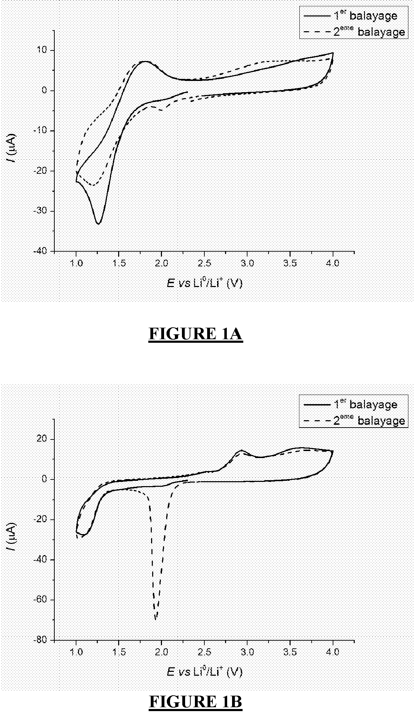

La variation du potentiel électrique appliquée à l'électrode de platine ou de carbone vitreux est mesurée (Figures 1A et 1B respectivement).

Sur l'électrode de platine (2mm) (Figure 1A), on observe le processus de réduction réversible du (1,4-benzene)-bisdithioate de bis-lithium à un potentiel de 1,25 V vs Li⁺/Li⁰ (courbe en trait plein) puis l'apparition d'un nouveau processus redox au 2^{ème} balayage (courbe pointillée) est détecté à 1,99 V.

Sur l'électrode de carbone vitreux (3 mm) (Figure 1B), la réduction irréversible du (1,4-benzene)-bisdithioate de bis-lithium est mesurée à un potentiel de 1,09 V vs Li⁺/Li⁰ (courbe en trait plein). Sur cette électrode, l'électro-génération d'un nouveau produit au 2^{ème} balayage (courbe pointillée) est observé à un potentiel de 1,93 V vs Li⁺/Li⁰.

### EXEMPLE 3

### Préparation d'une électrode

Une électrode ayant la composition BBDTLi₂:SuperP:Liant = 40:40:20 (en % massique) est préparée selon les étapes suivantes.

40 % de BBDTLi₂ et 40 % de SuperP sont broyés en présence de cyclohexane. Le cyclohexane est ensuite évaporé à température ambiante pendant 30 minutes.

20 % de liant polyvinyldifluoride (PvdF solution 12 %) et la N-méthyle-pyrrolidone (NMP) sont ensuite ajoutés afin de former une encre.

Cette encre est enduite avec une épaisseur de 200 µm sur un collecteur de courant en cuivre, puis est séchée dans une étuve à 55°C pendant 24 heures.

Des électrodes à diamètre de 14 mm sont découpées à l'aide d'un emporte-pièce puis séchées dans un Buchi à 80°C pendant 48 heures.

### EXEMPLE 4

### Fabrication d'une demi-pile bouton

Afin de déterminer les performances électrochimiques du matériau d'électrode selon l'invention, un accumulateur de type « pile bouton » est réalisé avec :
- une électrode négative de lithium métallique (1 M LiTFSI dans TEGDME:diox = 1:1 (% volumique)) ;
- une électrode positive constituée d'une composition BBDTLi₂:SuperP:Liant = 40:40:20 (% massiques), comprenant le composé de l'invention préparé selon l'exemple 1 déposé sur du cuivre; et
- deux séparateurs à base de polyoléfines.

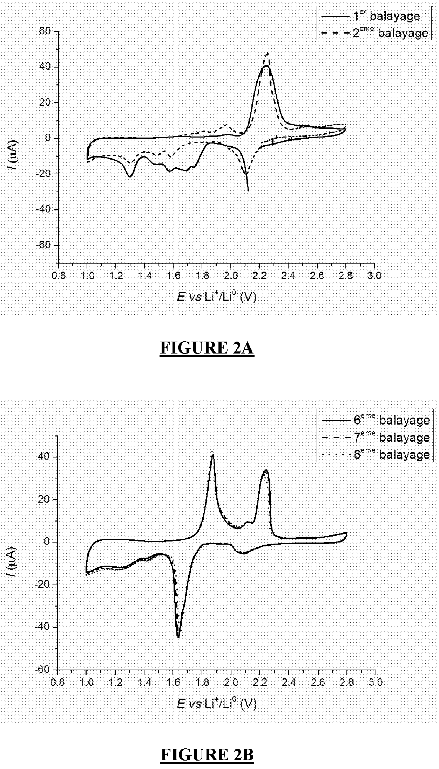

La variation du potentiel électrique au cours des cycles est mesurée (Figure 2A (1^{er} et 2^{ème} balayages) et Figure 2B (du 6^{ème} au 8^{ème} balayage)).

On observe au premier balayage, l'existence de plusieurs processus de réductions entre 1,2 et 1,8 V vs Li⁺/Li⁰ et dès le 2^{ème} cycle, on observe l'apparition de nouvelles signatures électrochimiques, pour générer ensuite un système très stable (Figure 2B), caractérisé par un processus de réduction à un potentiel de 1,64 V vs Li⁺/Li⁰ et deux processus d'oxydation à 1,86 et 2,23 V vs Li⁺/Li⁰.

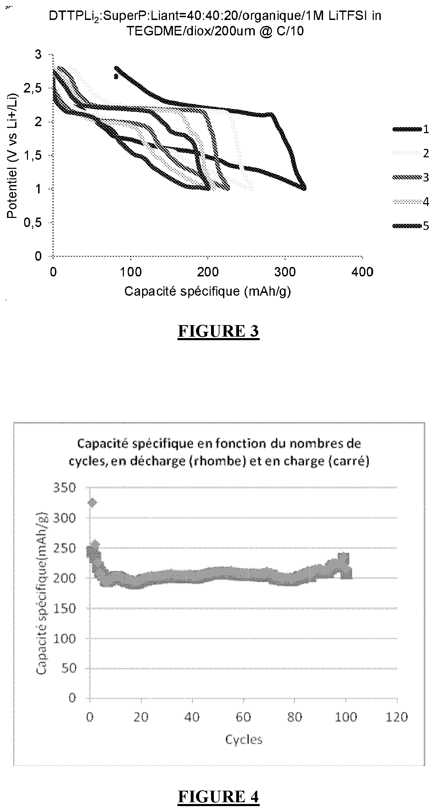

La capacité spécifique en fonction du potentiel électrique est également mesurée (Figure 3). Au premier cycle, une capacité de 320 mAhg⁻¹ supérieure à la capacité théorique du BBDTLi₂ (Qₜₕ = 221 mAhg⁻¹) est mesurée. Puis la capacité spécifique diminue et se stabilise à ∼200 mAhg⁻¹ après 5 cycles (Figure 4).

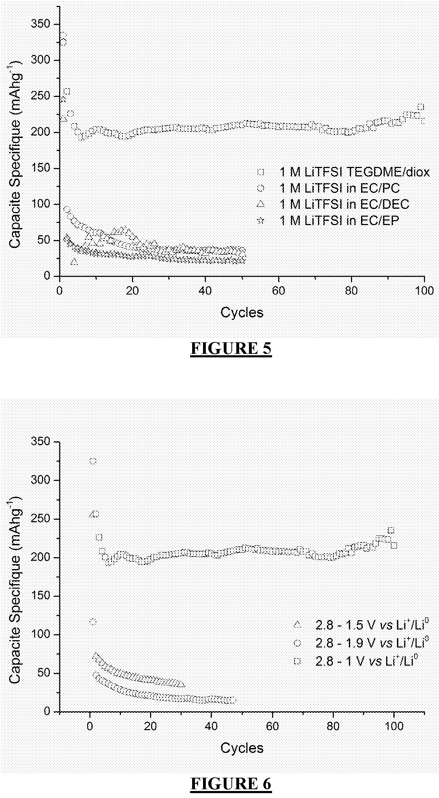

La tenue en cyclage du matériau électro-généré a été mesurée dans divers conditions, soit en changeant le solvant (Figure 5, de 1 à 1,8 V), en remplaçant le mélange d'éthers avec des mélanges carbonates, soit dans différents domaines de potentiel (Figure 6).

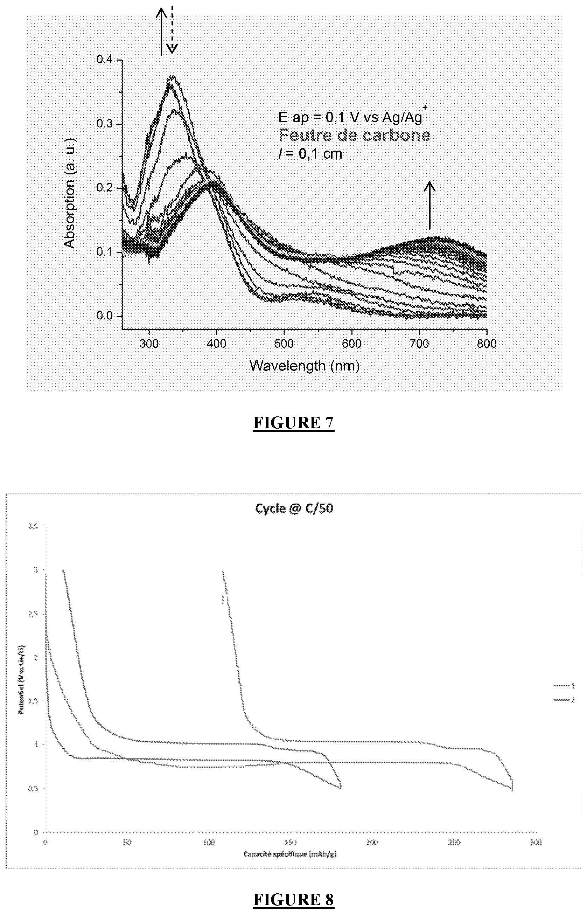

Afin de comprendre la nature du matériau électro-généré in situ pendant le cyclage du BBDTLi₂, des études spectro-électrochimiques ont été réalisées (Figure 7).

L'électrolyse exhaustive du BBDTLi₂ dans 0,1 M de tetrabutylammonium hexafluorophosphate dans l'acétonitrile (TBAPF₆/AN) suivie par spectroscopie UV-Vis entraine des changements spectroscopiques importantes comme la diminution en intensité de la bande à ∼340 nm accompagné par l'émergence de nouvelles bandes en proche IR.

### EXEMPLE 5

### Comparaison des performances du BBDTLi₂ avec le téréphtalate de dilithium TPLi₂ (non conforme)

Préparation du téréphtalate de dilithium (TPLi₂)
1 g d'acide téréphtalique est dispersé dans 50 mL d'un mélange éthanol/eau 1/1 en volume, puis 0,44 g de carbonate de lithium est ajouté.

La solution est chauffée dans un autoclave à 110 °C pendant 48 heures. Le précipité est ensuite séparé par centrifugation et lavé à l'éthanol puis séché à 50 °C. 1 g de poudre blanche est obtenu.

### Performances électrochimiques du TPLi₂

La caractérisation électrochimique du TPLi₂ est réalisée selon la procédure décrite dans les exemples 3 et 4 précédents, excepté le fait que l'électrolyte utilisé est une solution de LiPF₆ (1M) dans un mélange de carbonate, et que le régime de charge est de C/50.

La figure 8 montre l'évolution du potentiel en fonction de la capacité spécifique au cours des deux premiers cycles en régime C/50. Ces courbes montrent une diminution très significative de la capacité au cours des deux premiers cycles.

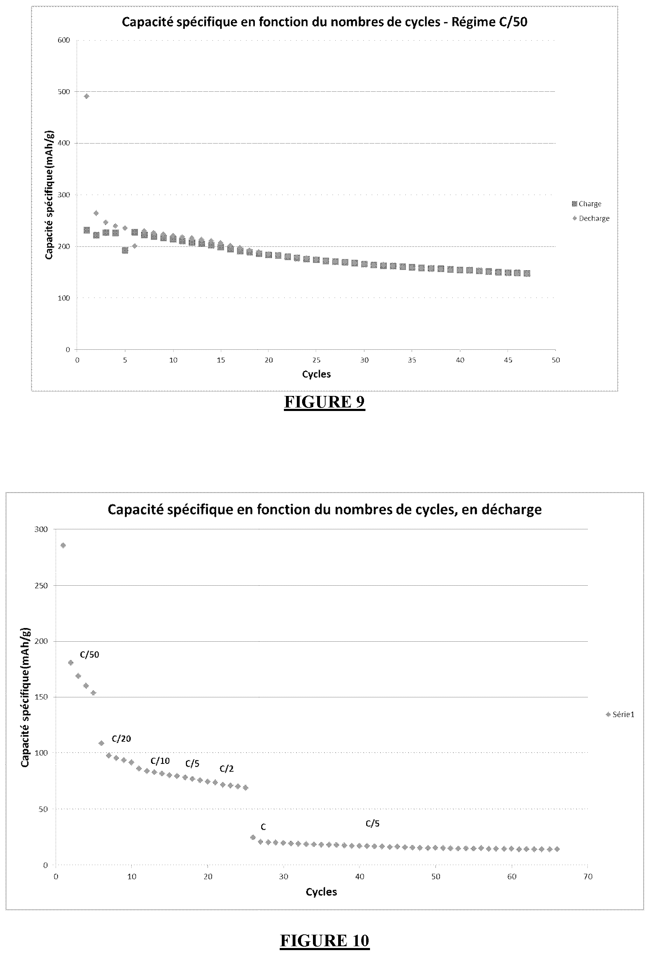

La mesure de la capacité spécifique en fonction du nombre de cycles, en régime C/50 (figure 9), montre une diminution constante de la capacité avec le nombre de cycles. Par ailleurs, il peut être noté, de la figure 10 représentant la capacité spécifique en fonction du nombre de cycles, en régime de décharge, que les capacités au régime C/50 sont supérieures à celles au régime C/10.

### Comparaison des performances du BBDTLi₂ et du TPLi₂

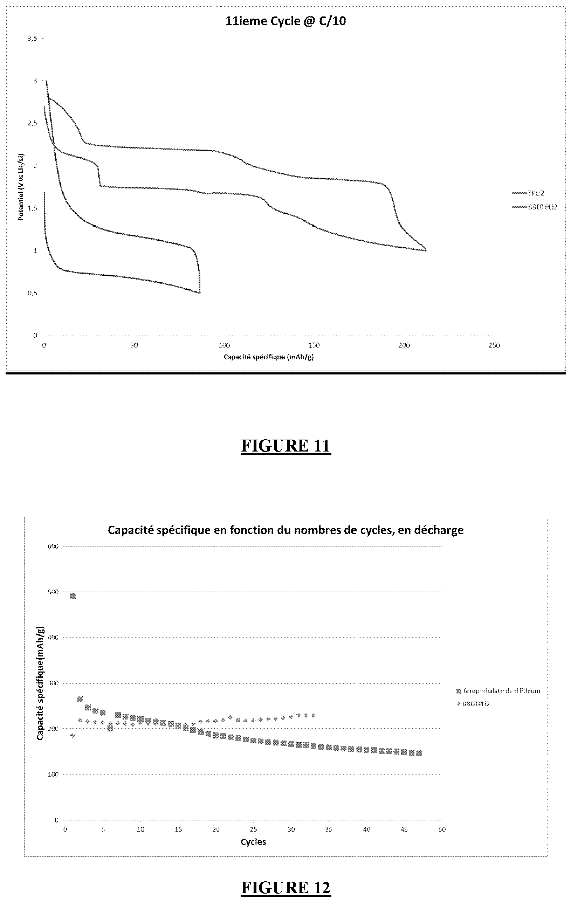

L'évolution du potentiel en fonction de la capacité spécifique, lors du 11^{ème} cycle en régime C/10, pour le BBDTLi₂ et le TPLi₂, est représentée en figure 11.

De même, sur la figure 12, les courbes ■ et ◆ représentent respectivement l'évolution de la capacité spécifique, en fonction du nombre de cycle, en décharge, pour l'accumulateur avec le TPLi₂ et celui avec le BBDTLi₂, en régime C/50 pour le premier et en régime C/10 pour le second.

Le tableau 1 suivant récapitule les performances des accumulateurs mettant en oeuvre le BBDTLi₂ conforme à l'invention et le TPLi₂ comme matériau actif de l'électrode positive.

**TABLEAU 1**

| **Matériau actif de l'électrode positive** | **Tension⁽¹⁾ (V vs Li+/Li)** | **Capacité en régime C/10⁽²⁾** | **Tenue en cyclage** | **Perte irréversible (%)⁽³⁾** |
|---|---|---|---|---|
| **BBDTLi₂ (selon l'invention)** | 2 | 220 | Stable | 35 |
| **TPLi₂ (préparé par les inventeurs)** | 0,8 | 80 | Perte de performances à chaque cycle | 60 |
| | | | | |

| | | | | |
|---|---|---|---|---|
| ⁽¹⁾ tension en décharge (valeur moyenne de tension sur le 11^{ème} cycle) ; ⁽²⁾ capacité au 11^{ème} cycle (régime C/10) ; ⁽³⁾ perte en capacité décharge/charge au 1^{er} cycle. | | | | |

## Revendications

1. Utilisation comme matériau actif d'électrode d'un composé comprenant au moins une entité de formule (I) : 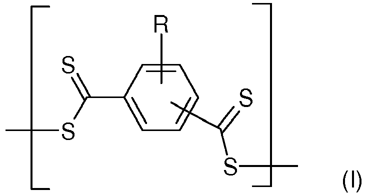 dans laquelle le groupement phénylique est substitué par un à quatre substituant(s) R, identique(s) ou différent(s), choisi(s) parmi un atome d'hydrogène, un atome d'halogène choisi parmi le fluor, le chlore, le brome ou l'iode, un groupement -C(=S)-S⁻C⁺, un groupement -O⁻C⁺, un groupement -S⁻C⁺, avec C⁺ étant un cation alcalin choisi parmi Li⁺, Na⁺ et K⁺, de préférence Li⁺; un radical (C₁-C₁₂)alkyle, un radical (C₂-C₁₂)alkényle, un radical (C₆-C₁₄)aryle ou hétéroaryle ; ou deux substituants R vicinaux pouvant le cas échéant être liés entre eux pour former ensemble un cycle de 3 à 7 chaînons; à l'état de base ou de sel ;
ainsi que ses formes tautomères, où dans le cadre de la présente invention un alkyle désigne un groupe aliphatique saturé linéaire, ramifié ou cyclique; un alkényle désigne un groupe aliphatique mono- ou polyinsaturé, linéaire, ramifié ou cyclique, comprenant une ou plusieurs insaturations éthyléniques; un aryle désigne un groupe aromatique mono-, bi- ou tricyclique; un hétéroaryle désigne un groupe hétérocyclique mono-, bi- ou tricyclique aromatique de 5 à 14 chaînons contenant de 1 à 8 hétéroatomes choisis parmi oxygène, le soufre et l'azote; un cycle de 3 à 7 chaînons désigne un groupe cyclique non aromatique, insaturé ou saturé, de 3 à 7 chaînons, comprenant éventuellement un ou plusieurs hétéroatomes choisi parmi l'oxygène, le soufre et l'azote.

2. Utilisation selon la revendication 1, **caractérisée en ce que** ledit composé vérifie la formule (II) : 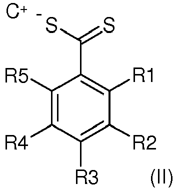 dans laquelle :
- C⁺ est un cation alcalin choisi parmi Li⁺, Na⁺ et K⁺, de préférence Li⁺; et
- au moins l'un des substituants R₁, R₂, R₃, R₄ ou R₅ représente un groupement - C(=S)-S⁻C⁺, C⁺ étant tel que défini précédemment ; les autres, identiques ou différents, représentant un substituant R tel que défini en revendication 1 ;
à l'état de base ou de sel ;
ainsi que ses formes tautomères.

3. Utilisation selon la revendication précédente, **caractérisée en ce que** R₃ représente un groupement -C(=S)-S⁻C⁺, et R₁, R₂, R₄ et R₅, identiques ou différents, représentent un substituant R tel que défini en revendication 1.

4. Utilisation selon l'une quelconque des revendications précédentes, **caractérisée en ce que** R représente un atome d'hydrogène, un groupement -C(=S)-S⁻C⁺, un groupement -O⁻C⁺ ou un groupement -S⁻C⁺, et de préférence R représente un atome d'hydrogène ; C⁺ étant un cation choisi parmi Li⁺, Na⁺ et K⁺, de préférence Li⁺.

5. Utilisation selon l'une quelconque des revendications précédentes, **caractérisée en ce que** le composé est le (1,4-benzene)-bisdithioate de bis-lithium de formule : 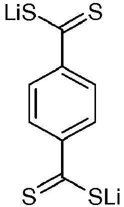 ou sa forme tautomère.

6. Composé de formule (IIa) : 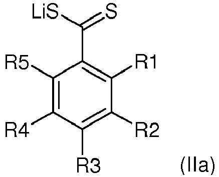 dans laquelle :
- au moins l'un des substituants R₁, R₂, R₃, R₄ ou R₅ représente un groupement -C(=S)-SLi ; les autres, identiques ou différents, représentant un substituant R choisi parmi un atome d'hydrogène, un atome d'halogène choisi parmi le chlore, le brome ou l'iode, un groupement -C(=S)-SLi, un groupement -OLi, un groupement -SLi, un radical (C₁-C₁₂)alkyle, un radical (C₂-C₁₂)alkényle, un radical (C₆-C₁₄)aryle ou hétéroaryle ; ou deux substituants R vicinaux pouvant le cas échéant être liés entre eux pour former ensemble un cycle de 3 à 7 chaînons ;
à l'état de base ou de sel ;
ainsi que ses formes tautomères, où dans le cadre de la présente invention un alkyle désigne un groupe aliphatique saturé linéaire, ramifié ou cyclique; un alkényle désigne un groupe aliphatique mono- ou polyinsaturé, linéaire, ramifié ou cyclique, comprenant une ou plusieurs insaturations éthyléniques; un aryle désigne un groupe aromatique mono-, bi- ou tricyclique; un hétéroaryle désigne un groupe hétérocyclique mono-, bi- ou tricyclique aromatique de 5 à 14 chaînons contenant de 1 à 8 hétéroatomes choisis parmi oxygène, le soufre et l'azote; un cycle de 3 à 7 chaînons désigne un groupe cyclique non aromatique, insaturé ou saturé, de 3 à 7 chaînons, comprenant éventuellement un ou plusieurs hétéroatomes choisi parmi oxygène, le soufre et l'azote.

7. Matériau actif d'électrode comprenant au moins un composé tel que défini en revendication 6.

8. Matériau selon la revendication 7, se présentant sous la forme d'une poudre dispersée ou en solution.

9. Electrode formée en tout ou partie d'un matériau actif comprenant au moins un composé tel que défini selon l'une quelconque des revendications 1 à 5, et au moins un additif conducteur électronique, de préférence choisi parmi des fibres de carbone, du noir de carbone, des nanotubes de carbone, du graphène et leurs analogues.

10. Electrode selon la revendication 9, comprenant en outre au moins un liant, de préférence choisi parmi des liants fluorés, en particulier parmi le polytétrafluoroéthylène, le polyfluorure de vinylidène, les polymères dérivés de carboxyméthylcellulose, les polysaccharides et les latex notamment de type caoutchouc styrène-butadiène.

11. Procédé de préparation d'une électrode telle que définie selon l'une quelconque des revendications 9 ou 10 comprenant au moins les étapes consistant en :
(i) disposer d'un mélange formé :
- d'au moins un composé tel que défini selon l'une quelconque des revendications 1 à 5,
- d'une phase liquide, en particulier comprenant un ou plusieurs solvants organiques ou aqueux,
- d'un ou plusieurs additif(s) conducteur(s) électronique(s), et
- éventuellement d'un ou plusieurs liant(s) ; et
(ii) déposer ledit mélange de l'étape (i) par enduction ou par une technique d'impression sur un substrat de base ou par extrusion ou par colaminage.

12. Procédé selon la revendication 11, ledit substrat de base étant un film polymérique de type polyéthylène ou polypropylène, ledit procédé comprenant une étape ultérieure (iii) de décollage dudit film polymérique, pour former une électrode autosupportée.

13. Procédé de préparation d'une électrode formée en tout ou partie d'un matériau actif comprenant au moins un composé tel que défini selon l'une quelconque des revendications 1 à 5, comprenant la formation d'un polymère par électropolymérisation *in situ* sur un collecteur de courant, de monomères fonctionnalisés par des entités de formule (I) telles que définies selon l'une quelconque des revendications 1 à 5.

14. Procédé selon la revendication 13, comprenant au moins les étapes consistant en :
(i) introduire lesdits monomères fonctionnalisés par des entités de formule (I) sous forme de poudre dans l'électrolyte d'une cellule électrochimique au lithium, sodium ou potassium, ou mettre en solution lesdits monomères fonctionnalisés dans l'électrolyte d'une cellule électrochimique au lithium, sodium ou potassium ; et
(ii) effectuer au moins un cycle de charge de la cellule pour former une électrode positive, ou au moins un cycle de décharge de la cellule pour former une électrode négative.

15. Batterie secondaire au lithium, au sodium ou au potassium, comprenant une électrode formée en tout ou partie d'un matériau actif comprenant au moins un composé tel que défini selon l'une quelconque des revendications 1 à 5.

16. Batterie selon la revendication précédente, **caractérisée en ce qu'**il s'agit d'une batterie secondaire au lithium, en particulier d'une batterie lithium-ion, lithium-polymère, lithium-soufre, lithium-air ou supercondensateur, et plus particulièrement d'une batterie lithium-ion.

## Patentansprüche

1. Verwendung, als aktives Elektrodenmaterial, einer Verbindung mit mindestens einer Einheit der Formel (I): 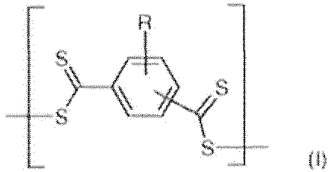 worin die Phenylgruppe mit einem bis vier identischen oder verschiedenen Substituenten R substituiert ist, die ausgewählt sind unter: aus einem Wasserstoffatom, einem Halogenatom, ausgewählt aus Fluor, Chlor, Brom oder Iod, einer -C(=S)-S⁻C⁺ -Gruppe, einer -O⁻C⁺ -Gruppe, einer -S⁻C⁺ -Gruppe, wobei C⁺ ein alkalisches Kation ist, ausgewählt unter Li⁺, Na⁺, und K⁺, vorzugsweise Li⁺, einem (C₁-C₁₂)-Alkylradikal, einem (C₂-C₁₂)-Alkenylradikal, einem (C₆-C₁₄)-Aryl- oder Heteroarylradikal; wobei zwei benachbarte Substituenten R gegebenenfalls miteinander verbunden sein können, um einen 3-bis 7-gliedrigen Ring zu bilden; im Zustand einer Base- oder eines Salzes; sowie ihre tautomeren Formen; wobei im Rahmen der vorliegenden Erfindung ein Alkyl eine gesättigte lineare aliphatische Gruppe bezeichnet, zyklisch oder verzweigt; ein Alkenyl eine einfach oder mehrfach ungesättigte lineare aliphatische Gruppe bezeichnet, zyklisch oder verzweigt, die eine oder mehrere ethylenische ungesättigte Stellen aufweist; ein Aryl eine mono-, bi-, oder trizyklische aromatische Gruppe bezeichnet; ein Heteroaryl eine heterozyklische mono-, bi-, oder trizyklische aromatische Gruppe mit 5 bis 14 Gliedern bezeichnet, die 1 bis 8 Heteroatome enthält, ausgewählt unter Sauerstoff, Schwefel und Stickstoff; ein 3- bis 7-gliedriger Ring eine nichtaromatische gesättigte oder ungesättigte zyklische Gruppe mit 3 bis 7 Gliedern bezeichnet, die gegebenenfalls ein oder mehrere Heteroatome enthält, ausgewählt unter Sauerstoff, Schwefel und Stickstoff.

2. Verwendung nach Anspruch 1, **dadurch gekennzeichnet, dass** die Verbindung die Formel (II) verfüllt: 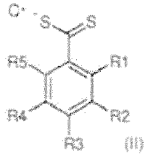 worin:
- C⁺ ein alkalisches Kation ist, ausgewählt unter Li⁺, Na⁺, und K⁺, vorzugsweise Li⁺; und
- mindestens einer der Substituenten R₁, R₂, R₃, R₄ oder R₅ eine -C(=S)-S⁻C⁺-Gruppe darstellt und C⁺ wie vorstehend definiert ist, die anderen Substituenten, gleich oder verschieden, einen Substituenten R darstellen wie in Anspruch 1 definiert; im Zustand einer Base- oder eines Salzes; sowie ihre tautomeren Formen.

3. Verwendung nach dem vorhergehenden Anspruch, **dadurch gekennzeichnet, dass** R₃ eine -C(=S)-S⁻C⁺-Gruppe repräsentiert und R₁, R₂, R₄ und R₅, gleich oder verschieden, einen Substituenten R repräsentieren wie in Anspruch 1 definiert.

4. Verwendung nach einem der vorhergehenden Ansprüche, **dadurch gekennzeichnet, dass** R ein Wasserstoffatom, eine -C(=S)-S⁻C⁺-Gruppe, eine -O⁻C⁺-Gruppe oder eine Gruppe -S⁻C⁺-Gruppe repräsentiert und R vorzugsweise ein Wasserstoffatom repräsentiert; wobei C⁺ ein Kation ist, ausgewählt unter Li⁺, Na⁺, und K⁺, vorzugsweise Li.

5. Verwendung nach einem der vorhergehenden Ansprüche, **dadurch gekennzeichnet, dass** die Verbindung das (1,4-Benzol)-Bisdithioat von bis-Lithium der Formel 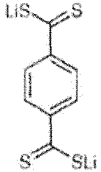 oder seine tautomere Form ist.

6. Verbindung mit der Formel IIa: 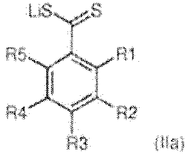 worin:
- mindestens einer der Substituenten R₁, R₂, R₃, R₄ oder R₅ eine -C(=S)-S^{Li}Gruppe darstellt; die anderen Substituenten, gleich oder verschieden, einen Substituenten R darstellen, der ausgewählt ist unter: aus einem Wasserstoffatom, einem Halogenatom, ausgewählt aus Chlor, Brom oder Iod, einer -C(=S)-SLi -Gruppe, einer -O^{Li} -Gruppe; einem (C₁-C₁₂)-Alkylradikal, einem (C₂-C₁₂)-Alkenylradikal, einem (C₆-C₁₄)-Aryl- oder Heteroarylradikal; wobei zwei benachbarte Substituenten R gegebenenfalls miteinander verbunden sein können, um einen 3- bis 7-gliedrigen Ring zu bilden, im Zustand einer Base- oder eines Salze; sowie ihre tautomeren Formen; wobei im Rahmen der vorliegenden Erfindung ein Alkyl eine gesättigte lineare aliphatische Gruppe bezeichnet, zyklisch oder verzweigt; ein Alkenyl eine einfach oder mehrfach ungesättigte lineare aliphatische Gruppe bezeichnet, zyklisch oder verzweigt, die eine oder mehrere ethylenische ungesättigte Stellen aufweist; ein Aryl eine mono-, bi-, oder trizyklische aromatische Gruppe bezeichnet; ein Heteroaryl eine heterozyklische mono-, bi-, oder trizyklische aromatische Gruppe mit 5 bis 14 Gliedern bezeichnet, die 1 bis 8 Heteroatome enthält, ausgewählt unter Sauerstoff, Schwefel und Stickstoff; ein 3- bis 7-gliedriger Ring eine nichtaromatische gesättigte oder ungesättigte zyklische Gruppe mit 3 bis 7 Gliedern bezeichnet, die gegebenenfalls ein oder mehrere Heteroatome enthält, ausgewählt unter Sauerstoff, Schwefel und Stickstoff.

7. Elektrodenaktivmaterial, das mindestens eine Verbindung nach Anspruch 6 enthält.

8. Material nach Anspruch 7, in der Form eines dispergierten Pulvers oder in Lösung.

9. Elektrode, die ganz oder zum Teil aus einer Verbindung gebildet ist, wie sie in einem der Ansprüche 1 bis 5 definiert ist, und mindestens einem elektronisch leitenden Additiv, vorzugsweise ausgewählt unter Karbonfasern, Ruß, Kohlenstoffnanotubes, Graphen und ihren Analoga.

10. Elektrode nach Anspruch 9, ferner umfassend mindestens ein Bindemittel, vorzugsweise ausgewählt unter fluorierten Bindemitteln, insbesondere aus Polytetrafluorethylen, Polyvinylidenfluorid, aus Carboxymethylcellulose abgeleiteten Polymeren, Polysacchariden und Latex, insbesondere des Typs Gummi Styrol-Butadien.

11. Verfahren zur Herstellung einer Elektrode gemäß der Definition nach einem der Ansprüche 9 bis 10, umfassend mindestens die Schritte, bestehend aus:
(i) Bereitstellen einer Mischung aus:
- mindestens einer Verbindung wie in einem der Ansprüche 1 bis 5 definert,
- einer flüssigen Phase, insbesondere 10, umfassend ein oder mehrere organische oder wässrige Lösungsmittel,
- einem oder mehreren elektronisch leitfähigen Zusatzstoffen und
- ggf. einem oder mehreren Bindemitteln; und
(ii) Aufbringen der Mischung aus Schritt (i) durch Beschichtung oder durch eine Drucktechnik auf einem Basissubstrat oder durch Extrusion oder durch Ko-Laminieren.

12. Verfahren nach Anspruch 11, bei dem das Basissubstrat eine Polymerfolie aus Polyethylen oder Polypropylen ist, wobei das Verfahren einen nachfolgenden Schritt (iii) des Abschälens der Polymerfolie umfasst, um eine selbsttragende Elektrode zu bilden.

13. Verfahren zur Herstellung einer Elektrode, die ganz oder zum Teil aus einem aktiven Material gebildet ist, das mindestens eine Verbindung nach einem der vorstehenden Ansprüche 1 bis 5 enthält, mit Bildung eines Polymers durch Elektropolymerisation in-situ auf einem Stromkollektor, aus Monomeren, die durch Struktureinheiten der Formel (I) funktionalisiert sind, wie in einem der Ansprüche 1 bis 5 definiert.

14. Verfahren nach Anspruch 13, umfassend mindestens die Schritte bestehend aus:
(i) Einführen der funktionalisierten Monomere mit Struktureinheiten der Formel (I) in Pulverform in den Elektrolyten einer elektrochemischen Lithium-, Natrium- oder Kalium-Zelle, oder Lösen funktionalisierten Monomere im Elektrolyten einer elektrochemischen Lithium-, Natrium- oder Kalium-Zelle; und
(ii) Ausführen mindestens eines Zelleladungszyklus, um eine positive Elektrode zu bilden, oder zumindest eines Zelleentladungszyklus, um eine negative Elektrode zu bilden.

15. Eine Lithium-, Natrium- oder Kalium-Sekundärbatterie, umfassend eine Elektrode, die ganz oder zum Teil aus einem aktiven Material gebildet ist, das mindestens eine Verbindung nach einem der vorstehenden Ansprüche 1 bis 5 enthält.

16. Batterie nach dem vorhergehenden Anspruch, **dadurch gekennzeichnet, dass** es eine Lithium-Sekundärbatterie ist, insbesondere eine Lithium-Ionen-Batterie, eine Lithium-Polymerbatterie, eine Lithium-Schwefel-Batterie, Lithium-Luft- oder Lithium-Supraleiter-Batterie, weiter insbesondere eine Lithium-Ionen-Batterie.

## Claims

1. Use as electrode active material of a compound comprising at least one entity of formula (I): 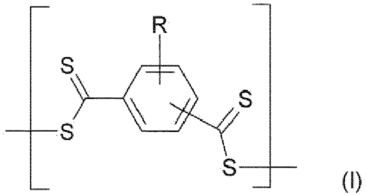 in which the phenyl group is substituted by one to four substituent(s) R, which are identical or different, chosen from a hydrogen atom, a halogen atom chosen from fluorine, chlorine, bromine or iodine, a -C(=S)-S⁻C⁺ group, an -O⁻C⁺ group, an -S⁻C⁺ group, with C⁺ being an alkali metal cation chosen from Li⁺, Na⁺ and K⁺, preferably Li⁺; a (C₁-C₁₂)alkyl radical, a (C₂-C₁₂)alkenyl radical or a (C₆-C₁₄)aryl or heteroaryl radical; or it being possible for two vicinal substituents R, if appropriate, to be bonded to one another in order to together form a 3- to 7-membered ring;
in the base or salt state;
and also its tautomeric forms,
wherein in the present invention an alkyl denotes a saturated and linear, branched or cyclic aliphatic group; an alkenyl denotes a mono- or polyunsaturated and linear, branched or cyclic aliphatic group comprising one or more ethylenic unsaturations; an aryl denotes a mono-, bi- or tricyclic aromatic group; a heteroaryl denotes a 5- to 14-membered mono-, bi- or tricyclic aromatic heterocyclic group containing from 1 to 8 heteroatoms chosen from oxygen, sulfur and nitrogen; a 3- to 7-membered ring denotes an unsaturated or saturated, nonaromatic, cyclic group optionally comprising one or more heteroatoms chosen from oxygen, sulfur and nitrogen.

2. Use as claimed in claim 1, **characterized in that** said compound confirms the formula (II): 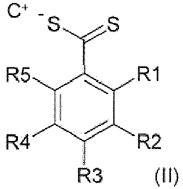 in which:
- C⁺ is an alkali metal cation chosen from Li⁺, Na⁺ and K⁺, preferably Li⁺; and
- at least one of the substituents R₁, R₂, R₃, R₄ or R₅ represents a-C(=S)-S⁻C⁺ group, C⁺ being as defined above, the others, which are identical or different, representing a substituent R as defined in claim 1;
in the base or salt state;
and also its tautomeric forms.

3. Use as claimed in the preceding claim, **characterized in that** R₃ represents a -C(=S)-S⁻C⁺ group, and R₁, R₂, R₄ and R₅, which are identical or different, represent a substituent R as defined in claim 1.

4. Use as claimed in any one of the preceding claims, **characterized in that** R represents a hydrogen atom, a -C(=S)-S⁻C⁺ group, an -O⁻C⁺ group or an -S⁻C⁺ group, and preferably R represents a hydrogen atom; C⁺ being a cation chosen from Li⁺, Na⁺ and K⁺, preferably Li⁺.

5. Use as claimed in any one of the preceding claims, **characterized in that** the compound is bislithium 1,4-benzenebisdithioate of formula: 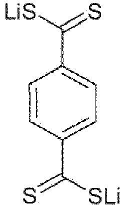 or its tautomeric form.

6. Compound of formula (IIa): 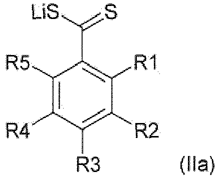 in which:
- at least one of the substituents R₁, R₂, R₃, R₄ or R₅ represents a -C(=S)-SLi group, the others, which are identical or different, representing a substituent R chosen from a hydrogen atom, a halogen atom chosen from chlorine, bromine or iodine, a -C(=S)-SLi group, an -OLi group, an -SLi group, a (C₁-C₁₂)alkyl radical, a (C₂-C₁₂)alkenyl radical or a (C₆-C₁₄)aryl or heteroaryl radical; or it being possible for two vicinal substituents R, if appropriate, to be bonded to one another in order to together form a 3- to 7-membered ring;
in the base or salt state;
and also its tautomeric forms,
wherein in the present invention an alkyl denotes a saturated and linear, branched or cyclic aliphatic group; an alkenyl denotes a mono- or polyunsaturated and linear, branched or cyclic aliphatic group comprising one or more ethylenic unsaturations; an aryl denotes a mono-, bi- or tricyclic aromatic group; a heteroaryl denotes a 5- to 14-membered mono-, bi- or tricyclic aromatic heterocyclic group containing from 1 to 8 heteroatoms chosen from oxygen, sulfur and nitrogen; a 3- to 7-membered ring denotes an unsaturated or saturated, nonaromatic, cyclic group optionally comprising one or more heteroatoms chosen from oxygen, sulfur and nitrogen.

7. Electrode active material comprising at least one compound as defined in claim 6.

8. Material as claimed in claim 7, which is provided in the form of a dispersed powder or in solution.

9. Electrode formed in all or part of an active material comprising at least one compound as defined according to any one of claims 1 to 5 and at least one electron-conducting additive, preferably chosen from carbon fibers, carbon black, carbon nanotubes, graphene and their analogues.

10. Electrode as claimed in claim 9, additionally comprising at least one binder, preferably chosen from fluorinated binders, in particular from polytetrafluoroethylene, polyvinylidene fluoride, polymers derived from carboxymethyl cellulose, polysaccharides and latexes, in particular of styrene-butadiene rubber type.

11. Process for the preparation of an electrode as defined according to any one of claims 9 or 10, comprising at least the stages consisting in:
(i) having available a mixture formed:
- of at least one compound as defined according to any one of claims 1 to 5,
- of a liquid phase, in particular comprising one or more organic or aqueous solvents,
- of one or more electron-conducting additive(s), and
- optionally of one or more binder(s); and
(ii) depositing said mixture from stage (i) by coating or by a printing technique on a base substrate or by extrusion or by corolling.

12. Process as claimed in claim 11, said base substrate being a polymeric film of polyethylene or polypropylene type, said process comprising a subsequent stage (iii) of detaching said polymeric film, in order to form a self-supported electrode.

13. Process for the preparation of an electrode formed in all or part of an active material comprising at least one compound as defined according to any one of claims 1 to 5, comprising the formation of a polymer by *in situ* electropolymerization on a current collector of monomers functionalized by entities of formula (I) as defined according to any one of claims 1 to 5.

14. Process as claimed in claim 13, comprising at least the stages consisting in:
(i) introducing said monomers functionalized by entities of the formula (I) in the powder form into the electrolyte of a lithium, sodium or potassium electrochemical cell or dissolving said functionalized monomers in the electrolyte of a lithium, sodium or potassium electrochemical cell; and
(ii) carrying out at least one charging cycle of the cell in order to form a positive electrode or at least one discharging cycle of the cell in order to form a negative electrode.

15. Lithium, sodium or potassium storage battery, comprising an electrode formed in all or part of an active material comprising at least one compound as defined according to any one of claims 1 to 5.

16. Battery as claimed in the preceding claim, **characterized in that** it is a lithium storage battery, in particular a lithium-ion, lithium-polymer, lithium-sulfur, lithium-air or supercapacitor battery and more particularly a lithium-ion battery.
